# EUROPÄISCHE PATENTSCHRIFT

(11) **EP 1 294 669 B1**
(45) Veröffentlichungstag und Bekanntmachung des Hinweises auf die Patenterteilung: **03.03.2004**
(21) Anmeldenummer: 01962789.2
(22) Anmeldetag: 27.06.2001
(51) Int. Cl.: C07C 45/50, C07C 47/02

(54) **VERFAHREN ZUR HYDROFORMYLIERUNG VON OLEFINEN MIT 2 BIS 8 KOHLENSTOFFATOMEN**
METHOD FOR THE HYDROFORMYLATION OF OLEFINS COMPRISING 2 TO 8 CARBON ATOMS
PROCEDE D'HYDROFORMYLATION D'OLEFINES AYANT 2 A 8 ATOMES DE CARBONE

(30) Priorität: 28.06.2000 DE 10031520
(43) Veröffentlichungstag der Anmeldung: 26.03.2003
(73) Patentinhaber: BASF AKTIENGESELLSCHAFT, 67056 Ludwigshafen (DE)
(72) Erfinder: RICHTER, Wolfgang, 67157 Wachenheim (DE); KROKOSZINSKI, Roland, 67273 Weisenheim a. Berg (DE); MÜLLER, Rolf, 67125 Dannstadt-Schauernheim (DE); MCATEE, Michael, US / Wilmington, N.C. 28412 (US)
(74) Vertreter: Kinzebach, Werner, Dr.
(86) Internationale Anmeldenummer: PCT/EP2001/007336
(87) Internationale Veröffentlichungsnummer: WO 2002/000582

(56) Entgegenhaltungen:
- DE-A- 19 530 698
- US-A- 4 827 043
- US-A- 5 648 553

## Beschreibung

Die vorliegende Erfindung betrifft ein Verfahren zur Hydroformylierung von Olefinen mit 2 bis 8 Kohlenstoffatomen.

Die Hydroformylierung oder Oxo-Synthese ist ein wichtiges großtechnisches Verfahren und dient der Herstellung von Aldehyden aus Olefinen und Synthesegas, d. h. einem Gemisch von Kohlenmonoxid und Wasserstoff. Die Hydroformylierung erfolgt in Anwesenheit von homogen im Reaktionsmedium gelösten Katalysatoren. Als Katalysatoren werden dabei im Allgemeinen Verbindungen oder Komplexe von Metallen der VIII. Nebengruppe, meist Co- oder Rh-Verbindungen bzw. -komplexe eingesetzt, die unmodifiziert oder z. B. mit aminoder phosphinhaltigen Verbindungen modifiziert sein können.

Bei der Hydroformylierung niederer Olefine ist ein nahezu vollständiger Umsatz des eingesetzten Olefins nur bei einem ausreichend groß bemessenen Reaktionsraum im Hydroformylierungsreaktor zu erreichen. Im Hinblick auf eine annehmbare Raum-Zeit-Ausbeute begnügt man sich in der Regel mit einem Teilumsatz, bezogen auf das eingesetzte Olefin. Aus dem Reaktionsaustrag wird das Hydroformylierungsprodukt abgetrennt, und die übrigen Bestandteile des Reaktionsaustrags werden zusammen mit frischem Kohlenmonoxid und Wasserstoff wieder dem Hydroformylierungsreaktor zugeführt. Mit dem zurückgeführten Strom werden allerdings auch die mit dem Synthesegas und/oder Olefin eingeführten gasförmigen Inertkomponenten, d. h. Gase, die der Hydroformylierung nicht zugänglich sind, wie Stickstoff, Methan, Argon usw., in den Reaktor zurückgeführt. Um zu verhindern, dass die Konzentration der Inertkomponenten im Hydroformylierungsreaktor kontinuierlich ansteigt und Werte erreicht, bei denen die Hydroformylierungsreaktion zum Erliegen kommt, muss laufend ein Teilstrom des zurückgeführten Stroms bzw. ein Teil der Gasphase im Hydroformylierungsreaktor aus dem Verfahren ausgeleitet werden, um die mit dem Synthesegas und/oder olefinhaltigen Zulauf eingeführten Inertkomponenten aus dem System zu entfernen.

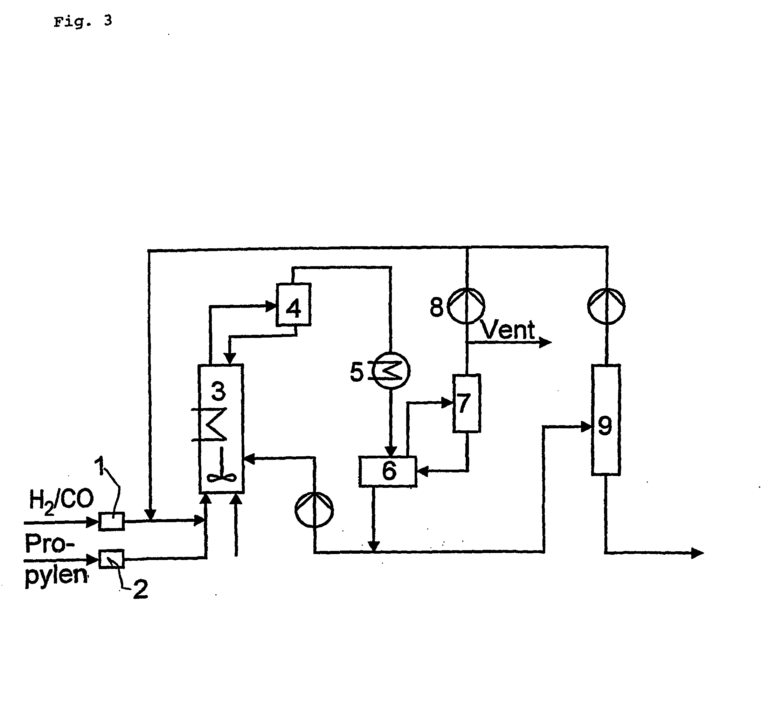

In M. Beller et al., Journal of Molecular Catalysis A: 104 (1995), 46-48 und der beiliegenden Fig. 3 ist ein typisches Kreisgasverfahren (gas recycle process) zur Hydroformylierung niederer Olefine dargestellt. Sorgfältig gereinigtes Propylen und Synthesegas (1,2) werden zusammen mit Kreisgas in den Reaktor (3) eingeführt. Der gasförmige Reaktoraustrag durchläuft einen Entnebler (4) zur Abscheidung mitgerissener Tröpfchen flüssiger Produkte. Das gasförmige Hydroformylierungsprodukt wird in einem Kühler (5) kondensiert und in einem Trenngefäß (6) gesammelt, aus dem das Kreisgas über einen Entnebler (7) und einen Kompressor (8) abgezogen und in den Reaktor (3) zurückgeführt wird. Ein Seiten- bzw. Ausleitstrom (Vent) wird abgeführt. Das rohe Hydroformylierungsprodukt aus dem Trenngefäß (6) wird der Aufarbeitung zugeführt.

Der Ausleitstrom enthält neben den Inertkomponenten erhebliche Mengen an nicht umgesetztem Olefin und anderer Wertkomponenten, die somit für die Umsetzung verloren gehen. Um den Ausleitstrom möglichst klein und die damit verbundenen Olefinverluste gering zu halten, wird im Allgemeinen ein Synthesegas hoher Reinheit eingesetzt. Dieses reine Synthesegas ist allerdings deutlich teurer als Synthesegase geringerer Reinheit. Synthesegase geringerer Reinheit, d. h. solche mit einem hohen Gehalt an Inertkomponenten, können in industriellen Hydroformylierungsverfahren aus den genannten Gründen nicht ohne Weiteres eingesetzt werden. Um zu verhindern, dass die Konzentration an Inertkomponenten im Hydroformylierungsreaktor unannehmbar hohe Werte erreicht, müsste der Ausleitstrom unter gleichzeitigem Verlust von unumgesetztem Olefin derart hoch gewählt werden, dass die Ersparnis des billigeren Einsatzstoffes zunichte gemacht würde.

Die JP 08092146-A beschreibt ein Verfahren zur Hydroformylierung von C₂-C₆-Olefinen. Das Abgas, das bei der destillativen Katalysatorabtrennung anfällt, wird komprimiert, um den darin enthaltenen Aldehyd zu verflüssigen.

Die JP 08208552-A beschreibt ein Hydroformylierungsverfahren, bei dem die anfallenden Abgasströme zur Rückgewinnung der darin enthaltenen Wertprodukte einer Wäsche mit den hochsiedenden Nebenprodukten der Hydroformylierung unterworfen werden.

Der vorliegenden Erfindung liegt die Aufgabe zugrunde, ein Verfahren zur Hydroformylierung von niederen Olefinen anzugeben, das den Einsatz von inertgashaltigem Synthesegas erlaubt, ohne dass wesentliche Mengen an Einsatzstoffen über den Ausleitstrom verloren gehen.

Diese Aufgabe wird erfindungsgemäß durch ein Verfahren zur Hydroformylierung von Olefinen mit 2 bis 8 Kohlenstoffatomen gelöst, bei dem man
a) in eine Reaktionszone einen olefinhaltigen Zulauf und ein Wasserstoff und Kohlenmonoxid enthaltendes Gasgemisch einspeist und in Gegenwart eines homogen im Reaktionsmedium gelösten Hydroformylierungskatalysators umsetzt,
b) den vom rohen Hydroformylierungsprodukt befreiten Reaktionsaustrag unter Ausschleusen eines gasförmigen Ausleitstroms in die Reaktionszone zurückführt, dadurch gekennzeichnet, dass
c) der Ausleitstrom zur Entfernung darin enthaltener nicht umgesetzter Olefine mit einer Waschflüssigkeit in innigen Kontakt gebracht wird, wobei es sich bei der Waschflüssigkeit um entgastes Hydroformylierungsprodukt handelt, und die mit Olefinen beladene Waschflüssigkeit zusammen mit dem rohen Hydroformylierungsprodukt der Aufarbeitung zugeführt wird.

Der gasförmige Ausleitstrom kann an einer beliebigen Stelle des aus Hydroformylierungsreaktor, Abtrennung des rohen Hydroformylierungsproduktes und Rückführung des vom Hydroformylierungsprodukt befreiten Reaktionsaustrages bestehenden Kreislaufs entnommen werden. Der Ausleitstrom kann z. B. aus der Gasphase der Reaktionszone entnommen werden. Zweckmäßigerweise wird ein Teilstrom des zurückgeführten, vom Hydroformylierungsprodukt befreiten Reaktionsaustrages abgezweigt und als Ausleitstrom ausgeschleust.

Die Größe des Ausleitstroms ist in der Regel so bemessen, dass damit im zeitlichen Mittel alle mit dem olefinhaltigen Zulauf und dem Synthesegas eingeführten gasförmigen Inertkomponenten, wie Stickstoff, Methan, Argon usw., aus dem System entfernt werden.

Der Ausleitstrom enthält - vor dem Inkontaktbringen mit der Waschflüssigkeit - in Abhängigkeit von der Stelle, an der er entnommen wird, wechselnde Mengen an nicht umgesetztem Olefin, Hydroformylierungsprodukt, gesättigten Kohlenwasserstoffen (insbesondere des dem eingesetzten Olefin entsprechenden Alkans) und gasförmigen Inertkomponenten, wie Stickstoff, Methan und Argon usw.

Der gasförmige Ausleitstrom wird, bevor er aus dem Verfahren ausgeschleust wird, zur Entfernung der darin enthaltenen nicht umgesetzten Olefine und gegebenenfalls anderer Wertprodukte, wie Hydroformylierungsprodukte oder Alkane, mit einer waschflüssigkeit in innigen Kontakt gebracht. Als Waschflüssigkeit wird erfindungsgemäß entgastes Hydroformylierungsprodukt, vorzugsweise das bei der Aufarbeitung des Reaktionsaustrages anfallende, von nicht umgesetzten Olefinen weitgehend befreite Hydroformylierungsprodukt, d. h. das gegenüber dem umgesetzten Olefin ein Kohlenstoffatom mehr aufweisende Alkanal und/oder Alkanol, verwendet. "Entgast" bedeutet für die vorliegenden Zwecke, dass das Hydroformylierungsprodukt unter den Druck- und Temperaturbedingungen, bei denen das Inkontaktbringen mit dem Ausleitstrom erfolgt, Olefine aufnehmen, d. h. lösen, kann. Mit anderen Worten soll "entgast" bedeuten, dass das als Waschflüssigkeit verwendete Alkanal und/oder Alkanol nicht Olefin-gesättigt ist. Die mit den Olefinen (und gegebenenfalls anderen Wertprodukten) beladene Waschflüssigkeit wird dann dem Aufarbeitungsteil des erfindungsgemäßen Verfahrens zugeführt.

Das Inkontaktbringen der Waschflüssigkeit mit dem gasförmigen Ausleitstrom kann in einer beliebigen zur Gaswäsche mit einer Flüssigkeit geeigneten Vorrichtung erfolgen. Zweckmäßigerweise erfolgt das Inkontaktbringen in einer Kolonne, wobei der Ausleitstrom in den Sumpf oder den unteren Teil der Kolonne eingeleitet und die Waschflüssigkeit am Kopf oder im oberen Teil der Kolonne eingeleitet und dem Ausleitstrom entgegengeführt wird. Vorzugsweise ist die Kolonne zur Schaffung einer großen Oberfläche mit Einbauten, wie Rieselböden, oder Füllkörpern, wie Raschig-Ringe, Spiralen oder Sattelkörpern, oder Einbauten versehen. Das Inkontaktbringen erfolgt auf diese Weise nach dem Gegenstromprinzip, wobei die beladene Waschflüssigkeit im unteren Teil der Kolonne und der gereinigte Ausleitstrom im oberen Teil der Kolonne abgezogen wird.

Das Inkontaktbringen von Ausleitstrom und Waschflüssigkeit erfolgt vorzugsweise unter Bedingungen, unter denen ein im Wesentlichen vollständiger Übertritt des im Ausleitstrom enthaltenen nicht umgesetzten Olefins und der gegebenenfalls enthaltenen weiteren Wertprodukte in die Waschflüssigkeit möglich ist. Durch die Absorption des nicht umgesetzten Olefins und gegebenenfalls weiterer Komponenten des Ausleitstroms in der Waschflüssigkeit wird Lösungswärme freigesetzt, die zu einer Erwärmung der Waschflüssigkeit führt. Wegen der bei Temperaturerhöhung abnehmenden Löslichkeit von Gasen in Flüssigkeiten nimmt die Aufnahmefähigkeit der Waschflüssigkeit mit zunehmender Temperatur ab. Es ist daher bevorzugt, die Waschflüssigkeit vor dem Inkontaktbringen mit dem Ausleitstrom auf eine Temperatur von weniger als 50 °C, insbesondere von weniger als 40 °C, zu kühlen. Bei Verwendung einer Kolonne zum Inkontaktbringen von Waschflüssigkeit und Ausleitstrom ist außerdem bevorzugt, die Kolonne und/oder die dem Ausleitstrom entgegengeführte Waschflüssigkeit aktiv zu kühlen. Hierzu sind z. B. in der Kolonne eingebaute Wärmetauscher geeignet, über die die Waschflüssigkeit herablaufen muss. Eine andere Möglichkeit der Kühlung besteht darin, die Waschflüssigkeit an einer oder mehreren Stellen der Kolonne zu sammeln und mittels einer Pumpe über einen außen liegenden Wärmetauscher und anschließend zurück in die Kolonne zu führen. Im Allgemeinen findet das Inkontaktbringen von Waschflüssigkeit und Ausleitstrom bei einer Temperatur von 20 bis 50 °C, vorzugsweise 25 bis 45 °C, statt.

Die relative Menge der Waschflüssigkeit, die mit dem Ausleitstrom in Kontakt gebracht wird, wird vorzugsweise so bemessen, dass bei der Temperatur, bei der das Inkontaktbringen erfolgt, ein möglichst vollständiger Übertritt der Wertprodukte in die Waschflüssigkeit erfolgt. Nach dem Abtrennen von der beladenen Waschflüssigkeit enthält der Ausleitstrom nach Maßgabe der Dampfdrücke des nicht umgesetzten Olefins, der gesättigten Kohlenwasserstoffe bzw. des Hydroformylierungsproduktes noch Wertkomponenten. Es kann daher vorteilhaft sein, den von der Waschflüssigkeit abgetrennten Ausleitstrom bis zur Kondensation des darin enthaltenen Olefins und/oder Hydroformylierungsprodukt, z. B. mittels eines Nachkühlers, zu kühlen und/oder zu komprimieren. Das kondensierte Olefin und/oder Hydroformylierungsprodukt kann aufgefangen und in das Verfahren zurückgeführt werden.

Der mit der Waschflüssigkeit behandelte Ausleitstrom enthält neben Inertgasen wesentliche Mengen, typischerweise 30 bis 70 Gew.-%, Kohlenmonoxid und Wasserstoff. Er kann z. B. thermisch verwertet werden oder als Einsatzstoff für Verfahren dienen, die Wasserstoff einsetzen und keine besonderen Anforderungen an dessen Reinheit stellen. So ist der Strom z. B. zur Herstellung von Methanol geeignet. Eine Übersicht über gängige Verfahren zur Herstellung von Methanol aus CO/H₂ enthaltenden Gasen findet sich in K. Weissermel und H.-J. Arpe, "Industrielle organische Chemie", VCH, 4. Auflage 1994, S. 31ff.

Olefine, die nach dem erfindungsgemäßen Verfahren hydroformyliert werden können, enthalten 2 bis 8 Kohlenstoffatome. Es kann sich um geradkettige, verzweigte oder cyclische Olefine handeln. Bevorzugte Beispiele geeigneter Olefine sind Ethen, Propen, 1-Buten und 2-Buten. Der olefinhaltige Zulauf kann ein einzelnes Olefin oder ein Gemisch von Olefinen enthalten. Das erfindungsgemäße Verfahren ist besonders geeignet für den Einsatz von Propylen unter Erzeugung von n- und iso-Butanal. Mit Vorteil ist jedoch auch der Einsatz von Ethylen unter Erzeugung von Propionaldehyd bzw. von 1-Buten unter Erzeugung von n- und iso-Valeraldehyd möglich. Der eingesetzte olefinhaltige Zulauf kann eine Beimischung eines gesättigten Kohlenwasserstoffs, in der Regel eines gesättigten Kohlenwasserstoffs gleicher Kohlenstoffzahl wie das eingesetzte Olefin, enthalten. Bei der erfindungsgemäßen Behandlung des Ausleitstroms mit der Waschflüssigkeit tritt neben dem nicht umgesetzten Olefin auch der gesättigte Kohlenwasserstoff weitgehend in die Waschflüssigkeit über und kann auf diese Weise in das Verfahren zurückgeführt werden. Um ein kontinuierliches Ansteigen der Konzentration des gesättigten Kohlenwasserstoffs in der Reaktionszone zu verhindern, ist es in der Regel erforderlich, an einer Stelle des Verfahrens eine Abtrennung des gesättigten Kohlenwasserstoffs vorzusehen. Zweckmäßigerweise kann das bei der Entgasung des rohen Hydroformylierungsprodukts anfallende Gemisch von nicht umgesetztem Olefin und gesättigtem Kohlenwasserstoff, z. B. destillativ, in eine Olefin-angereicherte und eine Olefinabgereicherte Fraktion getrennt werden, wobei lediglich die Olefin-angereicherte Fraktion in die Reaktionszone zurückgeführt wird.

Das eingesetzte Wasserstoff und Kohlenmonoxid enthaltende Gasgemisch wird üblicherweise als Synthesegas bezeichnet. Die Zusammensetzung des Synthesegases kann in weiten Bereichen variieren. Das molare Verhältnis von Kohlenmonoxid und Wasserstoff beträgt in der Regel 2:1 bis 1:2, insbesondere 45:55 bis 50:50. Die Vorteile des erfindungsgemäßen Verfahrens kommen besonders zum Tragen, wenn das Synthesegas Inertgase, wie Stickstoff, Methan oder Argon enthält, z. B. in einer Menge von 1 bis 15 Vol.-%.

Die Temperatur bei der Hydroformylierungsreaktion liegt im Allgemeinen in einem Bereich von 50 bis 200 °C, vorzugsweise etwa 60 bis 190 °C, insbesondere etwa 90 bis 190 °C. Die Umsetzung wird vorzugsweise bei einem Druck im Bereich von etwa 10 bis 700 bar, vorzugsweise 15 bis 200 bar, insbesondere 15 bis 60 bar, durchgeführt. Der Reaktionsdruck kann in Abhängigkeit von der Aktivität des eingesetzten Hydroformylierungskatalysators variiert werden.

In der Reaktionszone findet, bezogen auf das eingespeiste Olefin, pro Durchgang ein Teilumsatz statt. Der Umsatz beträgt im Allgemeinen 10 bis 90 % bezogen auf das eingespeiste Olefin.

Die Abtrennung des rohen Hydroformylierungsprodukts vom Austrag aus der Reaktionszone kann auf verschiedene Weise erfolgen. Es kann zum einen das sogenannte Flüssigaustragsverfahren zum Einsatz kommen, bei dem der im Wesentlichen, bis auf das im Überschuss zur Hydroformylierung eingesetzte Synthesegas und gasförmige Inertkomponenten, flüssige Austrag aus dem Hydroformylierungsreaktor in ein Entspannungsgefäß entspannt wird, wobei in Folge der Druckerniedrigung der Austrag in eine flüssige, den Katalysator, hochsiedende Nebenprodukte und geringe Mengen an Hydroformylierungsprodukt und an nicht umgesetztem Olefin enthaltende flüssige Phase und eine neben dem überschüssigen Synthesegas den Hauptteil des gebildeteten Hydroformylierungsproduktes und des nicht umgesetzten Olefins sowie die Inertkomponenten enthaltene Gasphase aufgetrennt wird. Die flüssige Phase kann zur Recyclierung des Katalysators als Rückführstrom wieder in die Reaktionszone geleitet werden. Die Gasphase wird abgezogen und z. B. einem Kondensator zugeführt, in welchem das rohe Hydroformylierungsprodukt flüssig abgeschieden wird. Die in dem Kondensator anfallende Gasphase, die im Wesentlichen nicht umgesetztes Synthesegas und nicht umgesetztes Olefin sowie Inertkomponenten enthält, wird ganz oder teilweise in die Reaktionszone zurückgeführt.

Mit Vorteil kann die in der Entspannungsstufe anfallende Gas- und Flüssigphase nach dem in der WO 97/07086 beschriebenen Verfahren aufgearbeitet werden. Zu diesem Zweck wird die Flüssigphase erwärmt und in den oberen Bereich einer Kolonne eingeleitet, während die Gasphase in den Sumpf der Kolonne eingeleitet wird. Flüssigphase und Gasphase werden dadurch im Gegenstrom geführt. Um den gegenseitigen Kontakt der Phasen zu erhöhen, ist die Kolonne vorzugsweise mit Füllkörpern gefüllt. Durch den innigen Kontakt der Gasphase mit der Flüssigphase werden die in der Flüssigphase vorhandenen Restmengen an Hydroformylierungsprodukt und nicht umgesetztem Olefin in die Gasphase transferiert, so dass der die Kolonne am Kopf verlassende Gasstrom im Vergleich zu dem am unteren Ende der Kolonne eingeführten Gasstrom an Hydroformylierungsprodukt und nicht umgesetztem Olefin angereichert ist. Die weitere Aufarbeitung des die Kolonne verlassenden Gasstroms und der die Kolonne verlassenden Flüssigphase erfolgt in üblicher Weise, beispielsweise wie oben beschrieben.

Alternativ kann man nach dem sogenannten Kreisgasverfahren verfahren, bei dem aus dem Gasraum der Reaktionszone ein Gasstrom abgezogen wird. Dieser Gasstrom besteht im Wesentlichen aus Synthesegas, unumgesetzten Olefinen und Inertkomponenten, wobei nach Maßgabe des Dampfdrucks des Hydroformylierungsproduktes in der Reaktionszone das bei der Hydroformylierungsreaktion gebildete Hydroformylierungsprodukt mitgeführt wird. Aus dem Gasstrom wird das mitgeführte Hydroformylierungsprodukt z. B. durch Abkühlen auskondensiert, und der vom Flüssiganteil befreite Gasstrom wird wieder zurück in die Reaktionszone geführt.

Das rohe Hydroformylierungsprodukt wird nach üblichem verfahren aufgearbeitet. Das rohe Hydroformylierungsprodukt enthält in der Regel wesentliche Mengen an gelöstem unumgesetzten Olefin und gegebenenfalls gesättigten Kohlenwasserstoffen. In einem ersten Aufarbeitungsschritt wird das rohe Hydroformylierungsprodukt daher vorzugsweise entgast. Das dabei anfallende entgaste Hydroformylierungsprodukt ist als Waschflüssigkeit im erfindungsgemäßen Verfahren geeignet. Zur Entgasung kann das rohe Hydroformylierungsprodukt entspannt, erwärmt und/oder mit einem Strippgas, wie Synthesegas oder Stickstoff, behandelt werden. Zweckmäßigerweise führt man die Entgasung in einer beheizten Kolonne durch, wobei das rohe Hydroformylierungsprodukt im Bereich der Kolonnenmitte eingespeist wird und das entgaste Hydroformylierungsprodukt aus dem Sumpf der Kolonne abgezogen wird. Am Kolonnenkopf fällt ein Gasstrom an, der im Wesentlichen aus nicht umgesetztem Olefin bzw. aus einem Gemisch von nicht umgesetztem Olefin und gesättigtem Kohlenwasserstoff besteht. Letzteres kann in seine Bestandteile Olefin und gesättigten Kohlenwasserstoff aufgetrennt werden. Das am Kolonnenkopf anfallende oder nach Auftrennung des anfallenden Gemischs erhaltene Olefin wird zweckmäßigerweise in die Reaktionszone zurückgeführt.

Geeignete Hydroformylierungskatalysatoren sind die üblichen, dem Fachmann bekannten Übergangsmetallverbindungen und -komplexe, die sowohl mit als auch ohne Cokatalysatoren eingesetzt werden können. Bevorzugt handelt es sich bei dem Übergangsmetall um ein Metall der VIII. Nebengruppe des Periodensystems und insbesondere um Co, Ru, Rh, Pd, Pt, Os oder Ir, speziell um Rh, Co, Ir oder Ru.

Es eignen sich z. B. Rhodiumkomplexe der allgemeinen Formel RhXₘL¹L²(L³)ₙ, worin
- X: für Halogenid, vorzugsweise Chlorid oder Bromid, Alkyl- oder Arylcarboxylat, Acetylacetonat, Aryl- oder Alkylsulfonat, insbesondere Phenylsulfonat und Toluolsulfonat, Hydrid oder das Diphenyltriazin-Anion,
- L¹, L², L³: unabhängig voneinander für CO, Olefine, Cycloolefine, vorzugsweise Cyclooctadien (COD), Dibenzophosphol, Benzonitril, PR₃ oder R₂P-A-PR₂, m für 1 oder 3 und n für 0, 1 oder 2 stehen. Unter R (die Reste R können gleich oder verschieden sein) sind Alkyl-, Cycloalkyl- und Arylreste zu verstehen, vorzugsweise Phenyl, p-Tolyl, m-Tolyl, p-Ethylphenyl, p-Cumyl, p-t-Butylphenyl, p-C₁-C₄-Alkoxyphenyl, vorzugsweise p-Anisyl, Xylyl, Mesityl, p-Hydroxyphenyl, das gegebenenfalls auch ethoxyliert vorliegen kann, Isopropyl, C₁-C₄-Alkoxy, Cyclopentyl oder Cyclohexyl. A steht für 1,2-Ethylen oder 1,3-Propylen. Bevorzugt stehen L¹, L² oder L³ unabhängig voneinander für CO, COD, P(Phenyl)₃, P(i-Propyl)₃, P(Anisyl)₃, P(OC₂H₅)₃, P(Cyclohexyl)₃, Dibenzophosphol oder Benzonitril.
- X: steht bevorzugt für Hydrid, Chlorid, Bromid, Acetat, Tosylat, Acetylacetonat oder das Diphenyltriazin-Anion, insbesondere für Hydrid, Chlorid oder Acetat.

Besonders bevorzugte Hydroformylierungskatalysatoren sind phosphorhaltige Rhodiumkatalysatoren, wie RhH(CO)₂(PPh₃)₂ oder RhH(CO)(PPh₃)₃·

Geeignete Hydroformylierungskatalysatoren werden z. B. in Beller et al., Journal of Molecular Catalysis A: 104 (1995), S. 17-85, beschrieben, worauf hier in vollem Umfang Bezug genommen wird.

Die Erfindung weist folgende Vorteile auf: Aus dem Ausleitstrom werden das nicht umgesetzte Olefin bzw. das Hydroformylierungsprodukt entfernt und in das Verfahren zurückgeführt. Dadurch entfällt die Notwendigkeit, den Ausleitstrom so gering wie möglich zu halten. Es kann daher ein Synthesegas mit deutlich höherem Gehalt an Inertkomponenten, wie Methan, Stickstoff, Argon usw., eingesetzt werden, das wesentlich kostengünstiger zu beziehen ist. Das erfindungsgemäße Verfahren eröffnet ferner die Möglichkeit, die Hydroformylierung bei einem höheren Olefinpartialdruck in der Reaktionszone durchzuführen. Es kann mit Vorteil ein Partialdruck des Olefins, insbesondere im Fall des Propylens, eingestellt werden, der 30 bis 40 % des Gesamtdrucks entspricht. Unter sonst gleichen Bedingungen führt der höhere Olefinpartialdruck in der Reaktionszone zu einer höheren Reaktionsgeschwindigkeit der Hydroformylierung. Andererseits kann die gleiche Reaktionsgeschwindigkeit bei einer niedrigeren Konzentration des Hydroformylierungskatalysators erreicht werden. Dieser Effekt kann mit Vorteil bei liganden-modifizierten Rhodiumkatalysatoren ausgenutzt werden. Es zeigt sich überraschenderweise, dass die Stabilität des Rhodiumkatalysators bei niedrigerer Konzentration höher ist als bei hohen Katalysatorkonzentrationen. Die Desaktivierung des aktiven Komplexes durch Ligandenabbau verläuft bei niedrigen Konzentrationen wesentlich langsamer. Geeignete Rhodiumkatalysator-Konzentrationen sind z. B. 50 bis 250 ppm Rhodium, bezogen auf die Flüssigphase in der Reaktionszone.

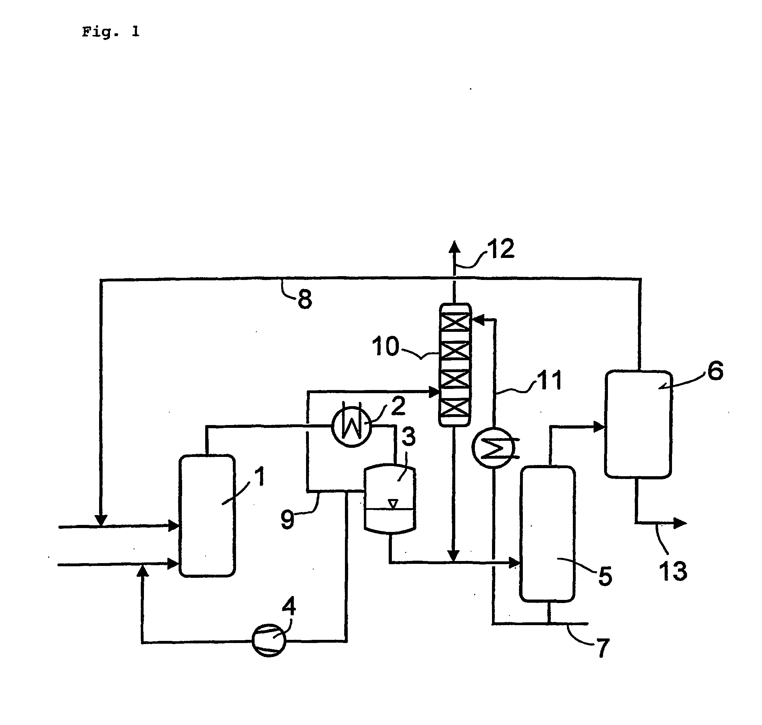

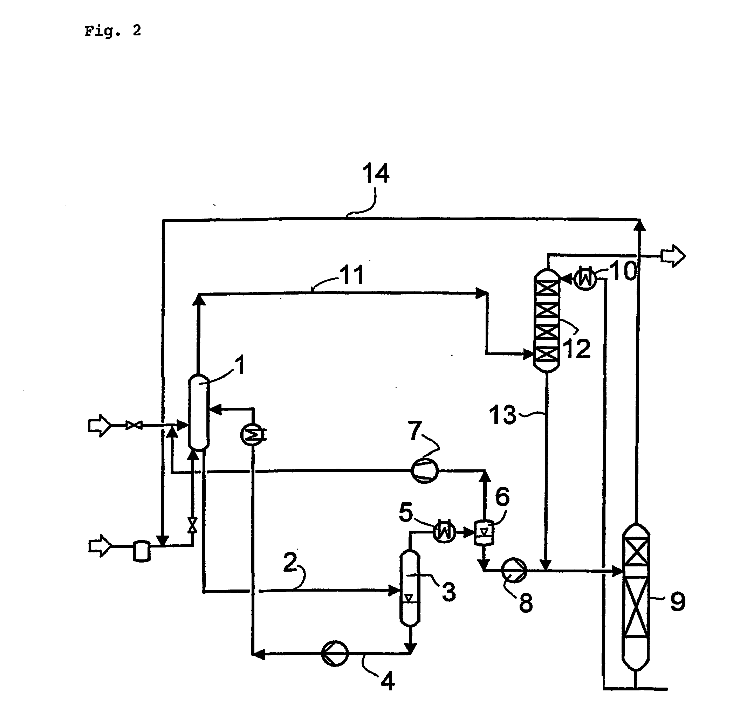

Vorteilhafte Ausgestaltungen des erfindungsgemäßen Verfahrens sind in den Fig. 1 und Fig. 2 dargestellt und werden im Folgenden erläutert. An sich selbstverständliche Anlagendetails, die zur Veranschaulichung des erfindungsgemäßen Verfahrens nicht erforderlich sind, wurden aus Gründen der Übersichtlichkeit weggelassen.

Fig. 1 zeigt eine schematische Darstellung des erfindungsgemäßen Verfahrens nach dem Kreisgasverfahren.

Fig. 2 zeigt eine schematische Darstellung des erfindungsgemäßen Verfahrens mit Flüssigaustrag.

Gemäß Fig. 1 wird in den Reaktor (1) ein olefinhaltiger Zulauf, der das zu hydroformylierende Olefin und einen gesättigten Kohlenwasserstoff enthält, und ein durch die Rohrleitung (8) zurückgeführter olefinhaltiger Strom sowie Synthesegas eingespeist und dort bis zu einem Teilumsatz hydroformyliert. Aus dem Gasraum des Reaktors wird ein gasförmiger Strom entnommen, der nicht umgesetztes Olefin, gesättigten Kohlenwasserstoff, nicht umgesetztes Synthesegas und ein Hydroformylierungsprodukt enthält. Der Strom wird im Wärmetauscher (2) gekühlt und einem Phasentrenngefäß (3) zugeführt. Der gasförmige Anteil wird teilweise über die Druckpumpe (4) wieder dem Reaktor (1) zugeführt. Der im Trenngefäß (3) anfallende flüssige Anteil, der im Wesentlichen aus rohem Hydroformylierungsprodukt mit darin gelöstem Olefin und gesättigtem Kohlenwasserstoff besteht, wird der Entgasungskolonne (5) zugeführt, an deren Kopf ein Gemisch von Olefin und gesättigtem Kohlenwasserstoff anfällt. Dieses Gemisch kann in der Rektifikationskolonne (6) in einen Olefin-angereicherten Strom, der über die Rohrleitung (8) in den Reaktor (1) zurückgeführt wird, und einen Olefin-abgereicherten Strom (13), der aus dem Verfahren ausgeschleust wird, getrennt werden. Im Sumpf der Entgasungskolonne (5) wird das rohe Hydroformylierungsprodukt entnommen, das anschließend weiter aufgearbeitet wird. Aus dem Kreisgassystem wird über die Leitung (9) ein Ausleitstrom abgezogen, über den die Inertgase ausgeschleust werden. Der Ausleitstrom wird in den unteren Teil der Kolonne (10) eingeleitet, wo er im Inneren der Kolonne hochsteigt. Am Kopf der Kolonne wird über die Leitung (11) entgastes Hydroformylierungsprodukt aus dem Sumpf der Entgasungskolonne (5) eingeleitet und dem aufsteigenden Ausleitstrom entgegengeführt. Das im Ausleitstrom enthaltene nicht umgesetzte Olefin und ein Großteil des Hydroformylierungsproduktes werden dadurch ausgewaschen und mit dem Sumpfaustrag der Kolonne (10) erneut der Entgasungskolonne (5) zugeführt. Zur Verbesserung der Aufnahmefähigkeit der Waschflüssigkeit kann die Absorptionswärme über an der Kolonne (10) angebrachte Wärmetauscher (nicht dargestellt) abgeführt und die Temperatur so z. B. unter 50 °C gehalten werden. Das über Leitung (12) entweichende Abgas der Kolonne (10) kann zur Kondensation restlicher Mengen an Olefin und Hydroformylierungsprodukt z. B. auf 0 °C abgekühlt werden. Das Kondensat kann abgeschieden und mit dem Sumpfaustrag der Kolonne (10) vereinigt werden.

Gemäß Fig. 2 werden in den Reaktor (1) das zu hydroformylierende Olefin und Synthesegas eingespeist und dort umgesetzt. Der Hydroformylierungsaustrag wird über die Rohrleitung (2) in das Entspannungsgefäß (3) entspannt, wobei sich in Folge der Druckabsenkung und Verdampfung der niedrig siedenden Komponenten eine flüssige Phase und eine Gasphase bildet. Die im Entspannungsgefäß (3) abgeschiedene flüssige Phase wird als flüssiger Strom über die Leitung (4) abgezogen und wieder in den Hydroformylierungsreaktor zurückgeführt. Der aus dem Gasraum des Entspannungsgefäßes (3) abgezogene, mit dem Hydroformylierungsprodukt und nicht umgesetztem Olefin angereicherte Gasstrom, der zusätzlich Inertkomponenten und gesättigte Kohlenwasserstoffe sowie nicht umgesetztes Synthesegas enthält, wird einem Kondensator (5) zugeführt, in dem seine höher siedenden Bestandteile - im Wesentlichen das Hydroformylierungsprodukt und ein Teil des nicht umgesetzten Olefins und der gesättigten Kohlenwasserstoffe - durch Kondensation abgetrennt werden.

Die aus dem Phasentrenngefäß (6) abgeführte Gasphase wird mittels des Verdichters (7) komprimiert und wieder in den Hydroformylierungsreaktor zurückgeführt. Die im Phasentrenngefäß (6) abgeschiedenen kondensierbaren Bestandteile werden über die Druckpumpe (8) der Entgasungskolonne (9) zugeführt. Am Kopf der Kolonne (9) fällt das nicht umgesetzte Olefin an, das über die Leitung (14) in den Hydroformylierungsreaktor zurückgeführt wird. Am Sumpf der Kolonne (9) wird entgastes Hydroformylierungsprodukt abgezogen, das zum Teil der weiteren Aufarbeitung zugeführt wird und zum Teil im Wärmetauscher (10) gekühlt wird und als Waschflüssigkeit zur Wäsche des über die Leitung (11) aus dem Hydroformylierungsreaktor (1) entnommenen Abgasstroms in der Waschkolonne (12) dient. Das am Kopf der Kolonne (12) anfallende Abgas wird aus dem System ausgeschleust. Die am Sumpf der Kolonne (12) anfallende, mit nicht umgesetztem Olefin beladene Waschflüssigkeit wird über die Leitung (13) der Entgasungskolonne (9) zugeführt.

Fig. 3 zeigt schematisch eine Anlage des Stands der Technik ohne Wäsche des Ausleitstroms (Vent).

Die Erfindung wird durch das folgende Beispiel näher veranschaulicht.

### Beispiel

Es wurde eine Anlage gemäß Fig. 1 verwendet. Dem Reaktor wurden ein Zulauf von 10 t/h 95 %iges Propylen (Rest Propan), ein Rückführstrom von 3,2 t/h aus der Rektifikationskolonne (6) sowie das für die Umsetzung notwendige Synthesegas zugeführt. Die Hydroformylierungsprodukte (n- und iso-Butyraldehyd) wurden zusammen mit dem nicht umgesetzten Propylen sowie dem zugeführten und dem bei der Reaktion gebildeten Propan mit Hilfe eines Kreisgasstroms aus dem Reaktor ausgetragen. Die kondensierbaren Anteile wurden in dem nachgeschalteten Kühler (2) auskondensiert und in dem darauf folgenden Abscheider (3) gesammelt. Die Flüssigphase enthielt 78,3 Gew.-% Butyraldehyd, 14,3 Gew.-% Propylen und 7,4 Gew.-% Propan. Diese Flüssigphase wurde der Entgasungskolonne (5) zugeführt (20,3 t/h), wo sie in ein von C₃-Kohlenwasserstofffreies rohes Oxoprodukt, das am Sumpf anfällt (15,9 t/h) und ein Gemisch aus 66 % Propylen und 34 % Propan, das am Kopf der Kolonne anfällt (4,4 t/h), aufgetrennt wurde. Das Propylen/Propan-Gemisch wird in der Kolonne (6) in einen praktisch propylenfreien Propanstrom am Sumpf (1,2 t/h) und ein Gemisch aus 90 % Propylen und 10 % Propan am Kopf (3,2 t/h) aufgetrennt. Dieser Strom wurde wieder dem Propylenzulauf des Reaktors (1) zugeführt. Aus dem Kreisgassystem wurde ein Teilstrom (6,5 t/h) (9) abgezogen und der Kolonne (10) zugeführt. Der Kreisgasstrom weist folgende Zusammensetzung auf:
- 51 Gew.-% Propan
- 32 Gew.-% Propylen
- 4 Gew.-% H₂
- 4 Gew.-% CO
- 3 Gew.-% Butyraldehyd
- 3 Gew.-% N₂
- 1 Gew.-% Methan

In den oberen Teil der Kolonne (10) wurden 19 t/h Butyraldehyd mit einer Temperatur von 35 °C aus dem Sumpf der Entgasungskolonne (5) eingeführt. Die Entgasungskolonne war mit vier Füllkörperbetten versehen. Durch zwei außenliegende Wärmetauscher wurde die Absorptionswärme abgeführt und die Temperatur bei 35 °C gehalten. Die im Ausleitstrom enthaltenen Wertstoffe Propylen, Propan und ein großer Teil des Butyraldehyds wurden ausgewaschen und mit dem Sumpfaustrag zurück in die Entgasungskolonne (5) (24,5 t/h) geführt. Das zurückgeführte Gemisch wies folgende Zusammensetzung auf: 78 Gew.-% Butyraldehyd, 14 Gew.-% Propylen, 8 Gew.-% Propan.

Das aus dem System ausgeleitete Abgas (0,9 t/h) wies folgende Zusammensetzung auf:
- 28 Gew.-% H₂
- 36 Gew.-% CO
- 26 Gew.-% N₂
- 6 Gew.-% CH₄
- 0,5 Gew.-% Propylen
- 0,03 Gew.-% Propan
- 3 Gew.-% Butyraldehyd

Es zeigt sich, dass das im Ausleitstrom enthaltene nicht umgesetzte Propylen durch die erfindungsgemäße Wäsche des Ausleitstroms weitgehendst in das Verfahren zurückgeführt wird.

## Patentansprüche

1. Verfahren zur Hydroformylierung von Olefinen mit 2 bis 8 Kohlenstoffatomen, bei dem man
a) in eine Reaktionszone einen olefinhaltigen Zulauf und ein Wasserstoff und Kohlenmonoxid enthaltendes Gasgemisch einspeist und in Gegenwart eines Hydroformylierungskatalysators umsetzt,
b) den vom rohen Hydroformylierungsprodukt befreiten Reaktionsaustrag unter Ausschleusen eines gasförmigen Ausleitstroms in die Reaktionszone zurückführt, **dadurch gekennzeichnet, dass**
c) der Ausleitstrom zur Entfernung darin enthaltener nicht umgesetzter Olefine mit einer Waschflüssigkeit in innigen Kontakt gebracht wird, wobei es sich bei der Waschflüssigkeit um entgastes Hydroformylierungsprodukt handelt, und die mit Olefinen beladene Waschflüssigkeit zusammen mit dem rohen Hydroformylierungsprodukt der Aufarbeitung zugeführt wird.

2. Verfahren nach Anspruch 1, **dadurch gekennzeichnet, dass** das Inkontaktbringen in einer Kolonne erfolgt, wobei der Ausleitstrom in den Sumpf oder den unteren Teil der Kolonne eingeleitet und die Waschflüssigkeit am Kopf oder im oberen Teil der Kolonne eingeleitet und dem Ausleitstrom entgegengeführt wird.

3. Verfahren nach Anspruch 1 oder 2, **dadurch gekennzeichnet, dass** das Kohlenmonoxid und Wasserstoff enthaltende Gasgemisch 1 bis 15 Vol.-% Inertgase enthält.

4. Verfahren nach einem der vorhergehenden Ansprüche, **dadurch gekennzeichnet, dass** die Waschflüssigkeit vor dem Inkontaktbringen mit dem Ausleitstrom auf eine Temperatur von weniger als 50 °C gekühlt wird.

5. Verfahren nach einem der Ansprüche 2 bis 4, **dadurch gekennzeichnet, dass** die Kolonne und/oder die dem Ausleitstrom entgegengeführte Waschflüssigkeit gekühlt wird.

6. Verfahren nach einem der vorhergehenden Ansprüche, **dadurch gekennzeichnet, dass** man als Olefin Propylen einsetzt und n- und iso-Butanal erzeugt.

7. Verfahren nach einem der vorhergehenden Ansprüche, **dadurch gekennzeichnet, dass** man als Hydroformylierungskatalysator einen phosphorhaltigen Rhodiumkatalysator verwendet.

8. Verfahren nach einem der vorhergehenden Ansprüche, **dadurch gekennzeichnet, dass** man den mit der Waschflüssigkeit behandelten Ausleitstrom bis zur Kondensation des darin enthaltenen Hydroformylierungsproduktes kühlt und/oder komprimiert.

9. Verfahren nach einem der vorhergehenden Ansprüche, **dadurch gekennzeichnet, dass** der Olefin-Partialdruck in der Reaktionszone 30 bis 40 % des Gesamtdrucks in der Reaktionszone beträgt.

10. Verfahren nach einem der vorhergehenden Ansprüche, **dadurch gekennzeichnet, dass** der mit der Waschflüssigkeit behandelte Ausleitstrom thermisch verwertet oder als Einsatzstoff für die Herstellung von Methanol verwendet wird.

## Claims

1. A process for the hydroformylation of olefins having from 2 to 8 carbon atoms, in which
a) an olefin-containing feed and a gas mixture comprising hydrogen and carbon monoxide are fed into a reaction zone and reacted in the presence of a hydroformylation catalyst,
b) the crude hydroformylation product is separated from the output stream from the reaction zone, a gaseous bleed stream is discharged and the remainder of the output stream is recirculated to the reaction zone, wherein
c) the bleed stream is brought into intimate contact with a scrubbing liquid to remove unreacted olefins present in the bleed stream, where the scrubbing liquid is degassed hydroformylation product, and the olefin-laden scrubbing liquid together with the crude hydroformylation product is passed to work-up.

2. A process as claimed in claim 1, wherein the bleed stream and the scrubbing liquid are brought into contact in a column in which the bleed stream is introduced into the bottom or the lower part of the column and the scrubbing liquid is introduced at the top or in the upper part of the column and is passed through the column in countercurrent to the bleed stream.

3. A process as claimed in claim 1 or 2, wherein the gas mixture comprising carbon monoxide and hydrogen contains from 1 to 15% by volume of inert gases.

4. A process as claimed in any of the preceding claims, wherein the scrubbing liquid is cooled to below 50°C before being brought into contact with the bleed stream.

5. A process as claimed in any of claims 2 to 4, wherein the column and/or the scrubbing liquid conveyed in countercurrent to the bleed stream are/is cooled.

6. A process as claimed in any of the preceding claims, wherein the olefin used is propylene and n-butanal and isobutanal are produced.

7. A process as claimed in any of the preceding claims, wherein the hydroformylation catalyst used is a phosphorus-containing rhodium catalyst.

8. A process as claimed in any of the preceding claims, wherein the bleed stream which has been treated with the scrubbing liquid is cooled and/or compressed until the hydroformylation product present therein condenses.

9. A process as claimed in any of the preceding claims, wherein the olefin partial pressure in the reaction zone is from 30 to 40% of the total pressure in the reaction zone.

10. A process as claimed in any of the preceding claims, wherein the bleed stream which has been treated with the scrubbing liquid is utilized to generate heat or is used as starting material for the preparation of methanol.

## Revendications

1. Procédé d'hydroformylation d'oléfines possédant 2 à 8 atomes de carbone, dans lequel on
a) introduit, dans une zone de réaction, une alimentation contenant l'oléfine et un mélange gazeux contenant de l'hydrogène et du monoxyde de carbone et on réalise la réaction en présence d'un catalyseur d'hydroformylation,
b) recycle le produit réactionnel libéré du produit d'hydroformylation brut, en soutirant un courant de sortie gazeux, dans la zone de réaction, **caractérisé en ce que**
c) le courant de sortie est intimement mélangé avec un liquide de lavage afin d'éliminer les oléfines n'ayant pas réagi contenues dans ce courant, le liquide de lavage étant le produit d'hydroformylation dégazé, et que l'on introduit le liquide de lavage chargé avec les oléfines conjointement avec le produit d'hydroformylation brut dans la zone de retraitement.

2. Procédé selon la revendication 1, **caractérisé en ce que** le mélange a lieu dans une colonne, le courant de sortie étant introduit dans le fond ou dans la partie inférieure de la colonne et le liquide de lavage étant introduit dans la tête ou dans la partie supérieure de la colonne et conduit à contre-courant par rapport au courant de sortie.

3. Procédé selon la revendication 1 ou 2, **caractérisé en ce que** le mélange gazeux contenant du monoxyde de carbone et de l'hydrogène contient 1% à 15% en volume de gaz inertes.

4. Procédé selon l'une quelconque des revendications précédentes, **caractérisé en ce que** le liquide de lavage est refroidi, avant le mélange avec le courant de sortie, à une température inférieure à 50°C.

5. Procédé selon l'une quelconque des revendications 2 à 4, **caractérisé en ce que** la colonne et/ou le liquide de lavage s'écoulant à contre-courant par rapport au courant de sortie sont refroidis.

6. Procédé selon l'une quelconque des revendications précédentes, **caractérisé en ce que** l'on met en oeuvre, en tant qu'oléfine, le propylène et **en ce que** l'on produit du n-butanal et de l'iso-butanal.

7. Procédé selon l'une quelconque des revendications précédentes, **caractérisé en ce que** l'on utilise, en tant que catalyseur d'hydroformylation, un catalyseur de rhodium contenant du phosphore.

8. Procédé selon l'une quelconque des revendications précédentes, **caractérisé en ce que** l'on refroidit et/ou l'on comprime le courant de sortie traité avec le liquide de lavage jusqu'à la condensation du produit d'hydroformylation contenu dans ce courant.

9. Procédé selon l'une quelconque des revendications précédentes, **caractérisé en ce que** la pression partielle de l'oléfine dans la zone de réaction représente 30% à 40% de la pression totale dans la zone de réaction.

10. Procédé selon l'une quelconque des revendications précédentes, **caractérisé en ce que** l'on utilise le courant de sortie traité avec le liquide de lavage pour produire de la chaleur ou qu'on l'utilise en tant que matière de départ pour la préparation de méthanol.
